# EUROPÄISCHE PATENTSCHRIFT

(11) **EP 0 585 617 B1**
(45) Veröffentlichungstag und Bekanntmachung des Hinweises auf die Patenterteilung: **15.10.1997**
(21) Anmeldenummer: 93112236.0
(22) Anmeldetag: 30.07.1993
(51) Int. Cl.: C12N 15/56, C12N 15/75, A21D 8/04, C12N 1/20

(54) **Bakterienxylanase, Verfahren zu ihrer Herstellung und ihre Verwendung bei der Herstellung von Brot**
Bacterial Xylanase, method for its production and its application in manufacturing bread
Xylanase bactérienne, méthode pour l'obtenir et son utilisation pour la fabrication de pain

(30) Priorität: 11.08.1992 DE 4226528
(43) Veröffentlichungstag der Anmeldung: 09.03.1994
(73) Patentinhaber: Röhm GmbH, 64293 Darmstadt (DE)
(72) Erfinder: Gottschalk, Michael, Dr., D-6105 Ober-Ramstadt (DE); Schuster, Erwin, Dr., D-6140 Bensheim-Auerbach (DE); Sprössler, Bruno, Dr., D-6101 Rossdorf (DE)

(56) Entgegenhaltungen:
- EP-A- 0 396 162
- ARCHIVES OF MICROBIOLOGY, Band 144, Nr. 3, April 1986; M.G. PAICE et al., Seiten 201-206

## Beschreibung

Die Erfindung betrifft eine neue Bakterien-Xylanase, ein Verfahren zur ihrer Herstellung sowie einen dafür geeigneten Bakterienstamm, ferner ein Plasmid mit dem zur Expression der neuen Xylanase geeigneten genetischen Material. Die Erfindung betrifft weiterhin ein Backmittel und ein Backverfahren zur Herstellung von Brot und Backwaren unter Verwendung der neuen Xylanase, wobei sie eine volumenerhöhende und teigverbessernde Wirkung entfaltet.

### Stand der Technik

Bei der Herstellung von Hefebackwaren wird ein hohes Volumen des Hefegebäckes, besonders bei Weißbrot und Brötchen, angestrebt. Das Backvolumen hängt von einer Reihe von Faktoren ab, die zum großen Teil in den Mehleigenschaften liegen. Aber auch die mit den besten Mehlen erzielbaren Backergebnisse lassen den Wunsch nach einem noch größeren Backvolumen offen. Die Suche nach volumensteigernden Backzusätzen hat einerseits zum Ziel, das zunächst erreichbare Backvolumen zu erhöhen, andererseits aber auch, mit minderen Mehlqualitäten wenigstens noch durchschnittliche Backergebnisse zu erzielen.

Zu den volumensteigernden Backzusätzen gehören Emulgatoren, Oxidantien, Reduktionsmittel, Hefeaktivatoren, Enzyme, wie Amylasen, Proteasen und Pentosanasen, pH-Stabilisatoren, Schleimstoffe, Fette usw.

G. Reed ("Enzymes in Food Processing", Academic Press, New York, London 1966, S. 253 ff.) hat festgestellt, daß unlösliche Pentosane im Mehl das Backvolumen herabsetzen. Er erhoffte sich daher gute Backergebnisse aus der Mitverwendung von Pentosanasen, die die Pentosane abbauen. Die mit derartigen Enzymen bisher beobachteten Effekte sind gering und nicht in allen Fällen reproduzierbar. A. Rotsch ("Brot und Gebäck", 1966, S. 91) fand, daß die Wirkung von Backemulgatoren durch die gleichzeitige Mitverwendung von Pentosanasen nicht oder nur in einem unbedeutenden Umfange erhöht werden kann, d.h. daß die Wirkung bei der gleichzeitigen Verwendung von Emulgatoren und Pentosanasen nicht additiv ist. Pentosanasen wurden jedoch gelegentlich zum Zwecke der Brotfrischhaltung vorgeschlagen. Cooke und Johannson (DT-AS 1 767 119) haben festgestellt, daß die Pentosanasen in dieser Hinsicht günstiger wirken als Backemulgatoren und statt ihrer verwendet werden sollten, jedoch wurde eine Wirkung auf das Backvolumen nicht gefunden.

Die Mitverwendung von Pentosanasen ist auch in Fällen bereits vorgeschlagen, in denen Roggenmehle ein ungünstiges Pentosan-Stärke-Verhältnis aufweisen, und zwar mit dem Effekt, daß der Teig solcher Mehle durch Nachquellen der Roggenpentosane eine zu hohe Viskosität besitzt. Beim Backen solcher Teige kommt es zum Abreißen der Krume von der Oberkruste, da der Teig einen hohen Stand erreicht und dieser im Ofen durch Ausbildung der Kruste fixiert wird. Der von der Kruste umschlossene Raum wird im Verlauf des Backvorganges nicht voll ausgefüllt, so daß es zu dem erwähnten Abreißen der Krume kommt. Durch den Zusatz von Pentosanasen werden die Pentosane abgebaut und die Teigviskosität entsprechend stark erniedrigt. Die Folge davon ist eine ausgeglichene Wasserbindung während des Backprozesses und eine Verhinderung des Abreißens der Krume von der Kruste. Bei der Verarbeitung von Mehlen mit einem ausgewogenen Verhältnis zwischen Pentosan und Stärke kann durch den Zusatz von Pentosanase die Viskosität des Teiges so weit herabgesetzt werden, daß flache Brote entstehen. Der Zusatz von Pentosanasen ist hier also nur in Ausnahmefällen von Vorteil, in der Mehrzahl der Fälle führt er jedoch zu Nachteilen. In Weizenmehlhefeteigen ist kein nennenswerter Effekt durch solche Pentosanasen erreichbar.

Kosmina beschreibt in "Biochemie der Brotherstellung", Leipzig 1975, S. 324, den nachteiligen Einfluß der sogenannten "Restfraktion" von Weizenmehl, die auch als Weizenschleimstoffe oder Tailings bezeichnet werden, wenn sie dem Mehl bei der Herstellung von Hefebackwaren aus Weizenmehl zugesetzt werden. Sie erhöhen zwar das Wasseraufnahmevermögen, verringern aber das Volumen und verschlechtern die Porung des Brotes. Diese nachteiligen Wirkungen lassen sich durch Vorbehandlung der "Restfraktion" mit einem Cellulasepräparat aus Trichoderma viride (Handelsprodukt "Meicelase" Meiji) aufheben.

Es ist weiterhin bekannt, das Volumen des Teiges und damit des Gebäcks durch den Zusatz von Enzymen, insbesondere von Amylasen und/oder Proteasen zu erhöhen. Zu dieser Maßnahme greift man insbesondere dann, wenn die Mehle einen zu geringen Eigengehalt an Enzymen aufweisen, jedoch ist die mit den genannten Enzymen erreichbare Volumenerhöhung gering. Auch der Zusatz von Backemulgatoren bewirkt eine gewisse Volumenerhöhung. Andere Zusätze, wie Hefeaktivatoren, Oxidantien und Reduktionsmittel, können zu einer weiteren Volumenzunahme führen.

Aus der DE-A 40 17 150 ist es bekannt, backaktive Pentosanase-Präparate zu erzeugen, indem man die Xylanasebildung von Mikroorganismen mit beta-Methylxylosid induziert. Dieses Verfahren kann bei Pilz- und Bakterienkulturen angewendet werden. Die Eigenschaften der gebildteten Xylanase hängen stark von der Wahl des erzeugenden Mikroorganismus ab.

Seit einiger Zeit werden Backhilfsmittel zur Herstellung von Hefebackwaren aus Weizenmehl eingesetzt, die eine über das bisher erreichbare Maß weit hinausgehende Volumensteigerung von Hefebackwaren aus Weizenmehl erreichen lassen. Neben Backemulgatoren und anderen Zusätzen enthält das erwähnte Backhilfsmittel ein hauptsätzlich aus Amylasen bestehendes Enzymgemisch, welches für die Volumensteigerung verantwortlich ist. Über die Charakteristik und die Gewinnung des Enzymgemisches ist nichts veröffentlicht. In dem Enzymgemisch läßt sich neben der Amylaseaktivität eine Xylanase-Aktivität nachweisen, deren Eigenschaftsspektrum sie als Pilz-Xylanase ausweist. Xylanasen gehören zur Enzymgruppe der Pentosanasen. Der damit bewirkte Volumeneffekt läßt sich mit üblichen bekannten Xylanasen, die sämtlich von Pilzen stammen, nicht annähernd erreichen.

Obwohl in letzter Zeit verschiedene Backenzyme auf Cellulasebasis angeboten worden sind, die eine deutliche Volumensteigerung von Hefebackwaren erreichen lassen, bleibt ihre Wirkung hinter derjenigen des erwähnten Backhilfsmittels weit zurück.

### Aufgabe und Lösung

Der Erfindung liegt die Aufgabe zugrunde, ein Backenzym zur Verfügung zu stellen, dessen Wirkung derjenigen des erwähnten Backhilfsmittels gleichkommt, und ein Verfahren zu seiner Herstellung aufzuzeigen, sowie ein Verfahren zur Herstellung von Hefebackwaren aus Weizenmehl mit entsprechend erhöhtem Volumen anzugeben.

Es wurde gefunden, daß sich aus bestimmten Bakterienkulturen Xylanasen mit den gewünschten Eigenschaften gewinnen lassen. Die Erfinder haben festgestellt, daß Bakterien-Xylanasen, die durch Expression des Strukturgens gemäß SEQ ID NO:2 herstellbar sind, beim Einsatz als Backenzym die erwünschte Wirkung entfalten.

Es wird vermutet, daß bei der Wirkung der neuen Xylanase ein partieller Abbau von Schleimstoffen des Mehles mit der Folge einer verbesserten Quellfähigkeit eine Rolle spielt, wobei der Abbau jedoch nicht zu einer Auflösung der unlöslichen Xylanpartikel führt. Ein Einfluß auf die Teigviskosität ist festzustellen: die Teige werden wollig und weich, was mit der Erhöhung des Backvolumens in einem ursächlichen Zusammenhang zu stehen scheint. Da jedoch das Viskositätsverhalten von Hefeteigen und das Backvolumen von vielfältigen Einflußgrößen abhängen, eignet es sich nur bedingt zur Charakterisierung der für die Erfindung wesentlichen Enzymwirkung. Dagegen kann die neue Xylanase durch ihre Aminosäuresequenz bzw. die entsprechende Nukleotidsequenz eindeutig identifiziert und von bekannten Xylanasen unterschieden werden.

Sie weicht von der bekannten B. subtilis-Xylanase nach G. Paice, R. Bourbonnais, M. Desrochers, L. Jurasek und M. Yaguchi (Arch. Microbiol. 1986, Vol.144, 201-206) in 45 Positionen der Nukleotidsequenz und in 10 Positionen der Aminosäuresequenz ab, die in SEQ ID NO:2 durch Unterstreichung hervorgehoben sind. Sie ist demnach als neue, bisher nicht beschriebene Xylanase anzusehen. Von den zehn abweichenden Aminosäuren befinden sich sechs im Anteil des reifen Proteins, das (nach Paice et al.) nach Abspaltung eines 28 Aminosäuren langen Leaderpeptids mit der Aminosäure 29 in SEQ ID NO:2 beginnt und 185 Aminosäuren umfaßt. Es wird angenommen, daß die einzigartige Backwirkung der neuen Xylanase auf den festgestellten Unterschieden in der Aminosäuresequenz - einzeln oder in ihrer Kombination - beruht.

Anlagen zur Beschreibung:
SEQ ID NO:1 enthält die gesamte aus RH1221 klonierte DNA-Sequenz. Darin befindet sich, beginnend mit dem Nukleotid 506, ein offener Leserahmen für 213 Aminosäuren.
SEQ ID NO:2 enthält das Strukturgen der erfindungsgemäßen Xylanase aus Bac. subtilis RH 1221 innerhalb SEQ ID NO:1 sowie die entsprechende Aminosäuresequenz dieser Xylanase. Dieses Strukturgen ist in dem Plasmid des Bac. subtilis RH 1330 pIK 91 mit der Hinterlegungsnummer DSM 7147 enthalten.

### Vorteile und Anwendungsgebiet der Erfindung

Die Bakterien-Xylanasen gemäß der vorliegenden Erfindung lassen eine Volumensteigerung von Hefebackwaren aus Weizenmehl um 20 bis 30 Vol.-%, zum Teil sogar darüber erreichen. Dieser Effekt wird - was bei Backmitteln sonst nicht beobachtet wird - bei allen Weizenmehlen erreicht, und zwar auch dann, wenn diese bereits durch Zusätze von Amylasen, Proteasen und Emulgatoren hinsichtlich des Backvolumens optimiert worden sind. Darüber hinaus wird die Teigbeschaffenheit verbessert und gelockert und die Bildung wolliger Teige erreicht. Die Porung des Gebäcks wird verfeinert.

Die Vorteile der Erfindung lassen sich bei der Herstellung aller Hefebackwaren auf Basis von Weizenmehl zur Geltung bringen. Größte Bedeutung hat die Erfindung für die Herstellung von Weißbrot und Brötchen. Sie wird jedoch auch bei der Herstellung von Milchbrötchen, Hefekuchen und Hefefeinbackwaren mit Vorteil angewandt.

### Die Bakterien-Xylanase

Die erfindungsgemäße Xylanase findet sich im Enzymspektrum von verschiedenen Bakterien. Durch übliche Auswahlverfahren lassen sich aus xylanasebildenden Mikroorganismen solche auswählen, die die erfindungsgemäße Xylanase in hoher Aktivität und Spezifität erzeugen. Ein Identifikation mit der erfindungsgemäßen Bakterien-Xylanase ist durch eine Analyse des entsprechenden Strukturgens des erzeugenden Bakteriums möglich; dafür stehen standardisierte Untersuchungsmethoden zur Verfügung. Hilfsweise kann die Transskription des Strukturgens in die entsprechende Peptidsequenz zur Identifikation der Xylanase selbst verwendet werden.

Bakterienstämme, die die neue Xylanase bilden, wurden in der Gattung Bacillus in den Arten Bac. subtilis, B. amyloliquefaciens, B. natto, B. mesentericus, B. licheniformis, B. stearothermophilus, B. coagulans, B. pumilus, B. megaterium, B. circulans und B. firmus gefunden. Wenn es auch möglich ist, aus dem Kulturfiltrat ausgewählter Bakterienstämme die gewünschte Xylanase zu gewinnen, so hat sich doch gezeigt, daß sie den für eine großtechnische Produktion gestellten Produktivitätsanforderungen nicht genügen. Mit den heute verfügbaren Methoden der Erbgutübertragung ist es jedoch grundsätzlich möglich, die für die Bildung der Xylanase maßgebliche Erbinformation aus einem der oben genannten Mikroorganismen auf andere, gut kultivierbare Mikroorganismen zu übertragen und das Enzym aus deren Kultur zu gewinnen. Dabei kann eine bedeutende Steigerung der Produktivität erreicht werden.

Ein in dieser Weise transformierter Bakterienstamm mit der Bezeichnung B. subtilis RH 1330 pIK 91 wurde unter der Hinterlegungsnummer DSM 7147 bei der Deutschen Sammlung von Mikroorganismen und Zellkulturen GmbH in Braunschweig entsprechend den Bestimmungen des Budapester Abkommens hinterlegt.

Bei Reihenuntersuchungen von Xylanasebildnern können geeignete Stämme gefunden werden. Das aus ihnen gewonnene Genom läßt sich mit gentechnischen Methoden auf bestimmte Plasmide übertragen, die sich leicht in mehreren Exemplaren in gut kultivierbare, toxikologisch unbedenkliche Bakterienstämme einbringen lassen. Das rekombinante Bakterium zeichnet sich dann durch eine hohe Produktivität der Xylanase aus. Die Züchtung der rekombinanten Bakterien unter geeigneten Kulturbedingungen führt zu Kulturbrühen oder -extrakten, aus denen sich die Xylanase beispielsweise durch Abtrennung der unlöslichen Zell- und Nährbodenbestandteile, Konzentration des Filtrats durch Ultrafiltration, Fällung durch Salze oder organische Flüssigkeiten oder Trocknung durch Versprühen, Wirbelschichttrocknung oder Lyophilisieren, gewinnen läßt. Die Xylanase wird bevorzugt in Granulat- bzw. Pulverform hergestellt und angewendet. Gegenüber Pilzkulturen haben die erfindungsgemäßen Bakterienkulturen den Vorteil, daß sie nur Fermentationszeiten von ein bis zwei Tagen benötigen, während bei Pilzen die Kulturdauer drei bis fünf Tage beträgt.

Charakteristisch für die erfindungsgemäße Xylanase ist ein Molekulargewicht von etwa 20.000 Dalton, während die Mehrzahl der bekannten Pilz-Xylanasen höhere Molekulargewichte haben. Diese bekannten Xylanasen bewirken keine vergleichbare Volumenerhöhung von Hefebackwaren. Die Aktivität des Enzyms gegenüber Xylan wird in Xylanase-Einheiten (UXyl) ausgedrückt, wobei 1 UXyl der Enzymmenge entspricht, welche aus Xylan bei 30^{o}C unter Standardbedingungen reduzierende Gruppen im Äquivalentwert von 1 µMol Xylose pro Minute freisetzt. Das pH-Optimum der neuen bakteriellen Xylanase liegt bei pH 6,0 und entspricht damit besser als Pilzxylanasen, die bei pH 4,5 - 5,0 optimal wirken, dem pH-Wert des Teiges, in dem sie ihre Wirkung entfalten soll.

Im Backversuch erweist sich die Volumen-Wirksamkeit der Xylanasen als weitgehend abhängig von der Dosierung. Bei geringen Dosierungen nimmt die Volumenerhöhung stets mit der eingesetzten Enzymmenge zu, jedoch wird bei einer bestimmten Enzymmenge ein Höchstwert der Volumensteigerung erreicht, der sich durch noch höhere Dosierungen nicht mehr übertreffen läßt. Häufig sinkt das Backvolumen bei erheblicher Überdosierung des Enzyms wieder ab, was sich durch einen fortschreitenden Abbau des Xylans erklären läßt. Die überraschende Wirksamkeit der erfindungsgemäßen Xylanase liegt vermutlich darin, daß sie bei geeigneter Dosierung verhältnismäßig schnell zu einem Aufschluß der zuvor unlöslichen, begrenzt quellfähigen Weizenschleimstoffe zu stark quellfähigen, jedoch immer noch ungelösten Partikeln führt, aber der weitere Abbau zu gelösten Hydrolyseprodukten langsam verläuft. Im Gegensatz dazu baut die Mehrzahl der bekannten Xylanasen die Weizenschleimstoffe fortlaufend bis zu löslichen Endprodukten ab, so daß die Zwischenstufe auf einem hohen Quellungsniveau nicht auftritt.

### Backmittel

Die erfindungsgemäße Xylanase wird vorzugsweise nicht als solche, sondern im Gemisch mit anderen Backzutaten als Backmittel eingesetzt. Dadurch wird die Dosierung und die gleichmäßige Vermischung mit den anderen Zutaten erleichtert.

Zum Abmischen des Enzyms zu einem Backmittel eignen sich grundsätzlich jede der zur Herstellung von Hefebackwaren gebräuchlichen Zutaten. Die Vermischung mit flüssigen Ausgangsstoffen, wie Wasser oder Milch, ist jedoch unzweckmäßig, ebenso die mit Hefe. Bevorzugte Abmischkomponenten sind Mehl, Zucker, Salz, Fette und Backemulgatoren. Die Abmischungen mit Mehl werden auch als Mehlverbesserungsmittel bezeichnet. Backemulgatoren werden bei der Herstellung von Hefebackwaren häufig zur Verbesserung der Krumenbeschaffenheit mitverwendet. Typische Beispiele sind Mono-, Di- und Triglyceride von Fettsäuren, Lecithin und Diacetylweinsäureester. Die bevorzugten Backmittel gemäß der Erfindung enthalten die Xylanase im Verschnitt mit derartigen Backemulgatoren. Ebenso können gewünschtenfalls weitere Backenzyme, wie Amylasen oder Proteasen in derartige Backmittel eingearbeitet werden. Die Xylanaseaktivität liegt in der Regel zwischen 0,1 und 10 UXyl/g des Backmittels. Es wird durch gleichmäßiges Vermischen des Xylanasepräparats mit dem Verschnittmittel, beispielsweise in einer Mischtrommel oder in einem Kneter, hergestellt.

Die Xylanase selbst ist bei Raumtemperatur über mehrere Jahre ohne nennenswerten Aktivitätsverlust lagerfähig. Backmittelformulierungen, die keine aktivitätsschädigenden Bestandteile, wie Proteasen, enthalten, haben eine vergleichbar gute Lagerfähigkeit. Der Wassergehalt sollte unter 5 %, bei Mitverwendung von Diacetylweinsäureemulgatoren unter 1 Gew.-% liegen.

### Backverfahren

Die Herstellung von Hefebackwaren aus Weizenmehl, Hefe, Wasser oder Milch, Fett, Salz, Zucker und gegebenenfalls weiteren gebräuchlichen Hilfsstoffen und Geschmackszusätzen ist allgemein bekannt. Die Mitverwendung der Backmittel gemäß der Erfindung bedingt keine Änderung der üblichen Backverfahren. Die für die Hefeentwicklung erforderliche Ruhezeit des Teiges ist ausreichend für die Einwirkung der Xylanase.

Zur Herstellung von Weißbrot und Brötchen wird das Backmittel im allgemeinen so dosiert, daß pro 100 kg Weizenmehl 200 bis 20.000 UXyl zur Anwendung kommen. Bei Pilzxylanasen liegt die Dosierung vorzugsweise zwischen 1000 und 10.000 UXyl und bei Bakterienxylanasen zwischen 200 und 2000 UXyl, jeweils pro 100 kg Mehl.

### BEISPIELE

### 1. Screening von Bacillusstämmen.

Aus Boden- und Wasserproben gewonnene oder aus öffentlichen Stammsammlungen bezogene B. subtilis-Bakterienstämme wurden zunächst auf einem Indikator-Nährboden aus Nutrient-Agar und einem Zusatz von 1 % feinverteiltem Xylan bei 37^{o}C 24 bis 48 Stunden gezüchtet. Stämme, die Xylanase bilden, erkennt man an den Hydrolyse-Höfen um die einzelnen Kolonien. Von 367 geprüften Bacillusstämmen konnten ca. 229 Xylan abbauen. Diese werden auf Schrägagar-Röhrchen überimpft und dienten als Stammkulturen für die im folgenden beschriebenen Versuche.

Die Stämme wurden in einer sterilen wäßrigen Nährlösung, enthaltend 6 % Sojamehl und 4 % Xylose bei pH 6,0 - 6,2 und 37^{o}C in Schüttelkolben 24 Stunden lang gezüchtet. Nach Abtrennung der Feststoffe bestimmt man in der wäßrigen Lösung die Xylanase-Aktivität bei pH 6,0, wo das pH-Optimum der Bakterien-Xylanase liegt. Dazu wurde ein Gemisch aus 0,75 ml Xylanlösung (Nutritional Biochemical Corporation, NBC, 0,5 %ig in 0,04 m Na-Acetatpuffer pH 6,0) und 0,25 ml Enzymlösung (in geeigneter Verdünnung) bei 30^{o}C 15 Minuten lang inkubiert. Die entstandenen reduzierenden Gruppen wurden danach nach der Methode von Somogyi und Nelson bestimmt (Lit.: Methods in Enzymology, Academic Press New York, London 1966 Vol. VIII, S. 7). Die Aktivität 1 UXyl entspricht der Enzymmenge, welche aus Xylan reduzierende Gruppen äquivalent 1µMol Xylose pro Minute bei 30^{o}C unter Standardbedingungen freisetzt.

Die Xylanasemenge wird aus einer Eichkurve abgelesen, die mit der Lösung reiner Xylose ermittelt wird. Die Enzymlösungen müssen so verdünnt werden, daß die Extinktionsdifferenzen zwischen Haupt- und Blindwert nicht über 0,25 Extinktionseinheiten liegen.

80 Stämme mit einer Xylanase-Aktivität von wenigstens 2 UXyl/ml Kulturlösung wurden gefunden. In einem zweiten Auswahlverfahren wurden die Kulturüberstände dieser Stämme im Backtest auf ihre volumenerhöhende Wirkung geprüft. Dabei wurden 8 Stämme gefunden, die erfindungsgemäße Xylanase in auswertbarer Menge erzeugen, während die anderen 72 Stämme eine Xylanase bilden, die keinen oder nur einen geringen Voulmeneffekt zeigen oder sogar volumenvermindernd wirken.

In der nachfolgenden Tabelle sind für sechs der erwähnten Stämme die Xylanase-Aktivität im Kulturüberstand und die Volumenerhöhung im Backversuch (Beispiel 8) angegeben.

| Stammbezeich- | Xylanase-Aktivität in UXyl/ml | Volumenerhöhung im Backversuch |
|---|---|---|
| RH 1225 | 3,5 | + 45 % |
| RH 1223 | 4,0 | + 36 % |
| RH 1221 | 6,0 | + 31 % |
| RH 1242 | 2,4 | + 37 % |
| RH 1205 | 11,3 | + 8 % |
| RH 1114 | 8,0 | - 10 % |

Einer der aus Beispiel 1 hervorgegangene Stämme mit hoher Xylanase-Aktivität, der der Art Bacillus subtilis zuzuordnen ist und sich durch gute Volumenerhöhung im Backversuch auszeichnet, wurde für die Herstellung des Enzyms im Laborfermenter ausgewählt und erhielt die Bezeichnung RH 1221.

Wie bei der Enzymentwicklung üblich, wurde aus dem Wildtyp-Stamm eine Variante selektiert, die die Fähigkeit zur Sporulation und zur Proteasebildung verloren hat. Dieser den Bedürfnissen der Produktion angepaßte Stamm wird im folgenden Beispiel 2 zur Enzymherstellung verwendet.

### 2. Herstellung der Bakterien-Xylanase im Laborfermenter.

Für die Zubereitung einer Nährlösung werden pro Liter eingesetzt:

| | |
|---|---|
| 60 g | Sojamehl |
| 40 g | Xylose |
| 13 g | Ammoniumhydrogenphosphat |
| 0,5 g | Magnesiumsulfat x 7 H₂O |
| 5 g | Calciumcarbonat |
| | Leitungswasser ad 1000 g. |

Die Nährbestandteile außer Xylose werden zusammengemischt. Der pH-Wert wird auf 6,0 - 6,2 eingestellt. Die Sterilisation erfolgt im Fermenter bei 121°C, 30 min unter Druck. Die Xylose-Lösung wird getrennt sterilisiert und nach dem Abkühlen zugegeben.

Zunächst wird eine Vorkultur angesetzt. Dazu werden in Schüttelkolben 400 ml obiger Nährlösung mit der Impföse aus der Schrägagarkultur nach Beispiel 1 beimpft und 10 Std. inkubiert.

Mit dem Inhalt dieser Vorkultur wird unter sterilen Bedingungen der Laborfermenter beimpft. Der Laborfermenter wird mit 8 l der oben beschriebenen Nährlösung gefüllt. Nach dem Beimpfen mit der Vorkultur wird mit einer Drehzahl von 700 U/min gerührt und mit 52 l Luft pro Stunde und Liter belüftet. Nach einer Inkubationszeit von 20 Stunden bei 37^{o}C wird die enzymhaltige Lösung durch Zentrifugation oder Filtration abgetrennt und auf Xylanase-Aktivität untersucht.

Die Proteinase-Aktivität wird ebenfalls bestimmt. Sie kann beim Backversuch zu starker Teigerweichung führen. Die Proteinase-Aktivität sollte daher möglichst gering sein.

In der geklärten Fermentationsbrühe wird eine Xylanase-Aktivität von 30 UXyl/g gemessen. Die Brühe wird zentrifugiert, durch Ultrafiltration konzentriert und schließlich lyophilisiert, so daß man ein pulverförmiges Enzympräparat mit einer Aktivität von 1500 UXyl/g erhält. Die Xylanase-Aktivität wird wie im Beispiel 1 bestimmt.

### 3. Gewinnung eines rekombinanten Bacillus subtilis-Stammes

Aus einem B. subtilis-Stamm mit der Bezeichnung RH 1321, der sich durch eine höhere Produktivität als RH 1221 auszeichnet, wurde chromosomale DNA nach der Methode von Marmur (Journ. Mol.Biol, Band 3, S.208, 1961) präpariert und partiell mit dem Restriktionsenzym Sau3A hydrolysiert. Die entstehenden DNA-Fragmente hatten eine Größenverteilung mit einem Maximum bei etwa 3000 bis 5000 Basenpaaren.

Das käufliche Plasmid pUB110 wurde vollständig mit dem Restriktionsenzym BamHI gespalten und mit dem oben erwähnten DNA-Fragmenten ein Ligationsansatz bereitet.

Dieser wurde in eine Xylase-negative Bac.subtilis-Mutante transformiert, die durch Mutation und Selektion aus einem käuflichen Stamm mit der Bezeichnung Bac.subtilis Marburg 168 H1 entwickelt worden war. Die Transformation wurde nach der Methode von Spizizen (Proc. Natl. Acad. Sci. 44, 1958, S. 1072) ausgeführt. Dabei werden Zellen, die Plasmid-DNA aufgenommen haben, durch das Antibiotikum Kanamycin selektiert, denn auf dem pUB110-Anteil der neu kombinierten Plasmide ist ein Kanamycin-Resistenzgen enthalten. Zur Selektion wurden die transformierten Zellen auf einem kanamycin-haltigen Nährboden vereinzelt, der durch einen zusätzlichen Xylangehalt die Xylanasebildung durch Trübung anzeigt.

Unter etwa 20 000 Kolonien, die durch Aufnahme des Plasmids kanamycin-resistent geworden waren, wurde eine Kolonie gefunden, die durch einen kleinen Hydrolysehof die Bildung von Xylanase anzeigte. Die Kolonie wurde isoliert, vermehrt und wieder auf dem Kanamycin/Xylan-Nährboden vereinzelt. Die Folgekolonien wiesen sämtlich einen Xylan-Hydrolysehof auf.

### 4. Sequenzierung des Xylanase-Gens aus RH 1221

Aus dem in Beispiel 3 erhaltenen Klon wurde ein Plasmid von etwa 6 kBp isoliert und als pIK91 bezeichnet. Darin wurde die in das Ausgangsplasmid pUB110 inserierte, aus RH 1221 stammende DNA nach der Methode von Sanger sequenziert. Die ermittelte Sequenz ist in der Anlage zur Beschreibung als SEQ ID NO:1 wiedergegeben. Auf den 1413 Basenpaaren wurde ein Open Reading Frame (eine für ein Protein codierende Sequenz) für 213 Aminosäuren gefunden, der in SEQ ID NO:2 angegeben ist.

### 5. Prüfung der exprimierten Xylanase im Backtest

Zur Prüfung der gemäß Beispiel 3 in B. subtilis Mb 168 H1 klonierten Bakterien-Xylanase wurden Schüttelkolbenkulturen angesetzt und der Kulturüberstand mit dem des Spenderstammes RH 1221 und dem des Wirtsstammes Mb 168 H1 verglichen. Dazu wurden Vorkulturen von je 5 ml Standard I-Bouillon in 100 ml-Erlenmeyerkolben ohne Schikane mit einer Impföse beimpft und bei 200 Upm und 37^{o}C im Rundschüttler mit 50 mm Schütteldurchmesser inkubiert. Beim erfindungsgemäß rekombinierten Stamm Mb 168 H1 pIK91 enthielt die Vorkultur zusätzlich 10 µg/ml Kanamycin. Von den Vorkulturen wurden je 15 ml Hauptkulturmedium (4 % Lactose, 6 % Sojamehl, 1,3 % (NH₄)₂HPO₄, 0,5 % CaCO₃, 0,05 5 MgSO₄ x 7 H₂O in Leitungswasser, pH 6,5) in 100 ml-Erlenmeyerkolben mit Schikane mit jeweils 150 µl Vorkultur beimpft und bei 200 Upm und 37^{o}C im Rundschüttler mit 50 mm Schütteldurchmesser inkubiert. Anschließend wurden die Zellen abzentrifugiert und die Kulturüberstände analysiert. Es wurden folgende Ergebnisse erhalten:

| Stamm | UXyl pH 6 | Backversuch |
|---|---|---|
| Spender RH 1221 | 10,6 | + 29 % |
| Wirt Mb 168 H1 Rekombin. | 0,0 | 0 % |
| Mb 168 H1 pIK91 | 2,8 | + 31 % |

Demnach erzeugt Mb 168 H1 pIK91 eine backaktive Xylanase, die wirkungsmäßig mit der Bakterien-Xylanase aus dem Stamm RH 1221 gleichwertig ist, wenn auch mit geringerer Produktivität.

### 6. Steigerung der Xylanase-Produktivität

Die Produktivität des Stammes B. subtilis 168 H1 pIK 91 reicht zur wirtschaftlichen Erzeugung der Bakterien-Xylanase nicht aus. Zur Gewinnung eines höher produktiven Stammes wurde eine Reihe hoch produktiver Xylanase-Stämme der Art B. subtilis nach der Methode von Chang und Cohen (Molec. gen. Genet. 168, 1979, S.111-115) mit pKI91 transformiert. Die pIK 91 enthaltenden Transformanten wurden wie unter Beispiel 5 beschrieben auf Xylanase-Produktivität geprüft. Zum weiteren Vergleich wurde ein technischer Produktionsstamm für alpha-Amylase mit der Bezeichnung BS22/17 transformiert und in gleicher Weise kultiviert und analysiert.

In den Kulturüberständen wurde folgende Xylanaseaktivitäten gefunden:

| Stamm | UXyl/g | Aktivität des plasmidfreien Wirtsstammes (UXyl/g) |
|---|---|---|
| RH 1321 pIK 91 | 15,2 | 10,2 |
| RH 1308 pIK 91 | 19,5 | 6,0 |
| RH 1330 pIK 91 | 57,8 | 8,5 |
| BS 22/17 pIK91 | 2,8 | 3,0 |

Dieses Ergebnis zeigt, daß die Produktivität des rekombinanten Stammes aus der Produktivität des Ausgangsstammes nicht vorhergesagt werden kann. Jedenfalls waren Stämme mit hoher Ausgangs-Produktivität nach Rekombination einem rekombinanten Stamm von geringerer Ausgangs-Produktivität deutlich unterlegen.

### 7. Bakterien-Xylanase-Herstellung mittels des rekombinanten Stammes B. subtilis RH 1330 pIK 91

7.1 Stammhaltung: Die Stammkultur wurde auf Schrägröhrchen wöchentlich überimpft, 24 Std. bei 37^{o}C inkubiert und bei 4^{o}C gelagert. Zusammensetzung des Schrägrohr-Nährbodens:

| | |
|---|---|
| NZ-Amin, Casein, enzymatisch hydrolysiert (Handelsbez. Sigma C 0626) | 10 g/l |
| Hefeextrakt (Handelsbez. Sigma C 0626) | 5 g/l |
| Kochsalz | 8 g/l |
| Agar | 10 g/l |
| dest. Wasser | |

pH 7,2 mit NaOH; Sterilisation 30 min 121^{o}C. Nach Abkühlen auf 45^{o}C werden 10 mg/l Kanamycin zugesetzt und sofort in Petrischalen ausgegossen.
7.2 Vorkultur: 1l-Erlenmeyer-Kolben mit Schikanen werden mit 100 ml Nährlösung folgender Zusammensetzung gefüllt:

| | |
|---|---|
| NZ-Amin, Casein, enzymatisch hydrolysiert (Handelsbez. Sigma C 0626) | 10 g/l |
| Hefeextrakt (Handelsbez. Sigma C 0626) | 5 g/l |
| Kochsalz | 8 g/l |

pH 7,2 mit NaOH; Sterilisation 30 min 121^{o}C. Nach Abkühlen auf 45^{o}C werden 10 mg/l Kanamycin zugesetzt. Die Nährlösung wird mit der Stammkultur 9.1 beimpft und 16 Std. bei 37^{o}C geschüttelt.
7.3 Vorfermenter: 8 l der in 7.2 verwendeten Nährlösung werden mit 500 ml der Vorkultur 7.2 beimpft. Kulturbedingungen: 37^{o}C, Belüftung 4,8 l/min, Rühren mit 700 Upm, 8 Std.
7.4 Hauptfermenter: 100 l einer Nährlösung aus

| | |
|---|---|
| Sojamehl | 80 g/l |
| Lactose | 60 g/l |
| Diammoniumhydrogenphosphat | 10 g/l |
| Magnesiumsulfat x 7 H₂O | 0,5 g/l |
| Calciumcarbonat | 5 g/l |

pH = 5,8 - 6,0, Sterilisation 30 min 121^{°}C.
Bei 37^{°}C wird mit 5 l der Vorfermenterkulturlösung 9.3 beimpft. Kulturbedingungen: 37^{°}C, Belüftung 60 l/min, Rühren mit 600 Upm, 30 Std. Die Kulturbrühe wird anschließend durch Filtration und Separation geklärt. Das Filtrat bzw. der Überstand wird durch Ultrafiltration konzentriert und lyophilisiert. Man erhält ca. 800 g Lyophilisat mit einer Aktivität von 15 000 UXyl/g.

### 8. Backversuch

Aus 100 Gew.-Tln. Mehl, 2 Gew.-Tln. Salz, 3 Gew.-Tln. Backhefe, 58 - 60 Gew.-Tln. Wasser und 40 - 50 ppm (bez. Teiggewicht) Ascorbinsäure wurde in einem Spiralkneter (Fabrikat Kemper) 2 - 3 min auf niedriger Stufe I und 3 - 4 min auf höherer Stufe II ein Teig bereitet. Dem Wasser wurden vor Beginn des Knetvorganges die in der Tabelle angegebenen Enzym-Mengen zugesetzt. Zum Vergleich mit der nach Beispiel 9 erzeugten Bakterien-Xylanase wurde das eingangs erwähnte Xylanase-haltige Enzymgemisch verwendet. Die Teigtemperatur betrug 25 - 27^{°}C. Nach einer Teigruhe von 20 min wurde der Teig zur Herstellung von freigeschobenem Weißbrot in 350 g-Stücke geteilt, geformt, 65 min bei 32^{°}C und 80 % rel. Luftfeuchte gegart und 30 min bei 230^{°}C gebacken. In der nachfolgenden Tabelle ist das Brotvolumen bei verschiedenen Enzymeinsätzen angegeben:

| | |
|---|---|
| ohne Enzym | 1264 - 1296 ccm |

Im Handel erhältliches Backenzym:

| | |
|---|---|
| 1500 UXyl/100 kg Mehl | 1304 ccm |
| 3000 UXyl/100 kg Mehl | 1304 ccm |
| 4500 UXyl/100 kg Mehl | 1372 ccm |

Erfindungsgemäße Bakterien-Xylanase nach Beispiel 7:

| | |
|---|---|
| 1500 UXyl/100 kg Mehl | 1392 ccm |
| 3000 UXyl/100 kg Mehl | 1468 ccm |
| 4500 Uxyl/100 kg Mehl | 1484 ccm |

Demnach werden mit den erfindungsgemäßen Bakterien-Xylanasen die Eigenschaften des im Handel erhältlichen Backenzyms nicht nur erreicht, sondern sogar übertroffen.

SEQ ID NO: 1

1 GGATCGTGACGGTCGGCTTGTCCGTTCTCGGTATCGTTCTTTTCATTGTC
CTCATGACGCTCGGCATCGTGGACGACGCGTTTAATAATATTGATGCCAA
101 TACATATTAAAAAGGAAAATCCCGGCGCATCCGCCCGGGATTTTTTCTTA
TTCCGCTTCTCCGACAACGGTAAAGTTTTTCTGCTTTGGATTTGCCAATA
201 CGGCGGCGAACAGCATTGACGTTGTATGCAGATATTCTTTCGACAAAAAA
GTTTTTTCAAATAAAACGTTTCCTCAGAGTTTCAACTGAAATCACTCAGT
301 TCTGCGAAATAAAACTATGGCGGTACAAGACCACTCATCATACAATTTTT
TGGGTCTTTGTAATTAAATTACAATTGTTCTTAATATTCACCCATGTATT
401 CATGCTATTATATTGAAAGGAACGATCAAAAGCGTTGGCGTTCGTTAAAT
ATTTACGAGTGCTGCCTCATGTCAAAGTCAGAAAAAATAGTATAGGAGGT
501 AACATATGTTTAAGTTTAAAAAGAAATTCTTAGTGGGATTAACGGCAGCT
TTCATGAGTATCAGCATGTTTTCGGCAACCGCCTCTGCAGCTGGCACAGA
601 TTACTGGCAAAATTGGACTGACGGGGGCGGGACAGTAAACGCAGTCAATG
GCTCTGGCGGAAATTACAGTGTTAATTGGTCTAATACCGGGAATTTCGTT
701 GTTGGTAAAGGCTGGACTACAGGCTCGCCATTTAGAACAATAAACTATAA
TGCCGGTGTTTGGGCGCCGAATGGCAATGGATATTTGACTTTATATGGCT
801 GGACGAGATCGCCCCTCATCGAATATTATGTGGTGGATTCATGGGGTACT
TACAGACCTACCGGAACGTATAAAGGTACCGTAAAGAGTGATGGAGGTAC
901 ATATGACATATATACAACGACACGTTATAACGCACCTTCCATTGATGGCG
ATAACACTACTTTTACGCAGTACTGGAGTGTCCGCCAGTCGAAGAGACCG
1001 ACCGGAAGCAACGCTGCAATCACTTTCAGCAATCATGTTAACGCATGGAA
GAGCCATGGAATGAATCTGGGCAGTAATTGGGCTTATCAAGTCTTAGCGA
1101 CAGAAGGATATAAAAGCAGCGGAAGTTCTAATGTAACAGTGTGGTAACAG
CTCATCTCTAATGAGGGGCAGCTAACGGGCTGCTGATCGTTCCTTGAGAA
1201 ATTTTATAATGATTGTGAAGAGGCGAACGGTTTTGGCATTACCGGAGGCA
GGATATTCTCCATCAGTTTTTTTAGCGCGTATTAAAATAAGAGTTCCGTC
1301 TTTTTTTGGTAACCCGCTTACAACAGACACCTTCACATAAGCCTTAAGCA
AAATAAAAATATCCCGGTTACCACCTCCTTTAGTTTCGGAGGTTTTCTTC
1401 ATTAAACAGATCC

SEQ IS NO: 2

506/1 536/11
ATG TTT AAG TTT AAA AAG AAA TTC TTA GTG GGA TTA ACG GCA GCT TTC ATG AGT ATC AGC
Met phe lys phe lys lys lys phe leu val gly leu thr ala ala phe met ser ile ser
566/21 596/31
ATG TTT TCG GCA ACC GCC TCT GCA GCT GGC ACA GAT TAC TGG CAA AAT TGG ACT GAC GGG
met phe ser ala thr ala ser ala ala gly thr asp tyr trp gln asn trp thr asp gly
626/41 656/51
GGC GGG ACA GTA AAC GCA GTC AAT GGC TCT GGC GGA AAT TAC AGT GTT AAT TGG TCT AAT
gly gly thr val asn ala val asn gly ser gly gly asn tyr ser val asn trp ser asn
686/61 716/71
ACC GGG AAT TTC GTT GTT GGT AAA GGC TGG ACT ACA GGC TCG CCA TTT AGA ACA ATA AAC
thr gly asn phe val val gly lys gly trp thr thr gly ser pro phe arg thr ile asn
746/81 776/91
TAT AAT GCC GGT GTT TGG GCG CCG AAT GGC AAT GGA TAT TTG ACT TTA TAT GGC TGG ACG
tyr asn ala gly val trp ala pro asn gly asn gly tyr leu thr leu tyr gly trp thr
806/101 836/111
AGA TCG CCC CTC ATC GAA TAT TAT GTG GTG GAT TCA TGG GGT ACT TAC AGA CCT ACC GGA
arg ser pro leu ile glu tyr tyr val val asp ser trp gly thr tyr arg pro thr gly
866/121 896/131
ACG TAT AAA GGT ACC GTA AAG AGT GAT GGA GGT ACA TAT GAC ATA TAT ACA ACG ACA CGT
thr tyr lys gly thr val lys ser asp gly gly thr tyr asp ile tyr thr thr thr arg
926/141 956/151
TAT AAC GCA CCT TCC ATT GAT GGC GAT AAC ACT ACT TTT ACG CAG TAC TGG AGT GTC CGC
tyr asn ala pro ser ile asp gly asp asn thr thr phe thr gln tyr trp ser val arg
986/161 1016/171
CAG TCG AAG AGA CCG ACC GGA AGC AAC GCT GCA ATC ACT TTC AGC AAT CAT GTT AAC GCA
gln ser lys arg pro thr gly ser asn ala ala ile thr phe ser asn his val asn ala
1046/181 1076/191
TGG AAG AGC CAT GGA ATG AAT CTG GGC AGT AAT TGG GCT TAT CAA GTC TTA GCG ACA GAA
trp lys ser his gly met asn leu gly ser asn trp ala tyr gln val leu ala thr glu
1106/201 1136/211
GGA TAT AAA AGC AGC GGA AGT TCT AAT GTA ACA GTG TGG TAA
gly tyr lys ser ser gly ser ser asn val thr val trp OCH

## Patentansprüche

1. Bakterien-Xylanase, dadurch gekennzeichnet, daß sie durch Expression des Strukturgens gemäß SEQ ID NO:2 herstellbar ist.

2. Bakterien-Xylanase, dadurch gekennzeichnet, daß sie durch Expression des Genoms des Plasmids herstellbar ist, das in dem Bacillus subtilis mit der Hinterlegungsnummer DSM 7147 enthalten ist.

3. Plasmid pIK 91, enthalten in dem Bacillus subtilis mit der Hinterlegungsnummer DSM 7147, auf dem das Strukturgen der SEQ ID NO:2, die zugehörige Leaderpeptidsequenz sowie die zur Genexpression in Bacillus subtilis erforderlichen DNA-Sequenzen kodiert sind.

4. Bakterien-Xylanase erzeugender Bakterien-Stamm, insbesondere der Art Bacillus subtilis, gekennzeichnet durch den Gehalt eines Strukturgens gemäß SEQ ID NO:2.

5. Bakterien-Xylanase erzeugender Bakterien-Stamm, insbesondere der Art Bacillus subtilis, gekennzeichnet durch den Gehalt eines Plasmids gemäß Anspruch 3.

6. Verfahren zur Herstellung einer Bakterien-Xylanase gemäß Anspruch 1 oder 2, dadurch gekennzeichnet, daß man ein Bakterium, das das Strukturgen gemäß SEQ ID NO:2 enthält, in üblicher Weise kultiviert und aus dem Kulturfiltrat die gebildete Bakterien-Xylanase gewinnt.

7. Verfahren nach Anspruch 6, dadurch gekennzeichnet, daß man ein Bakterium, das ein Plasmid gemäß Anspruch 3 enthält, in üblicher Weise kultiviert und aus dem Kulturfiltrat die gebildete Bakterien-Xylanase gewinnt.

8. Verfahren nach Anspruch 6 oder 7, dadurch gekennzeichnet, daß man ein Bakterium der Gattung Bacillus einsetzt.

9. Verfahren nach Anspruch 8, dadurch gekennzeichnet, daß man ein Bakterium der Art Bacillus subtilis einsetzt.

10. Verfahren nach Anspruch 9, dadurch gekennzeichnet, daß man den Bacillus subtilis mit der Hinterlegungsnummer DSM 7147 einsetzt.

11. Verwendung der Bakterien-Xylanase gemäß Anspruch 1 oder 2 zur Herstellung von Brot und Backwaren.

12. Backmittel zur Herstellung von Brot und Backwaren, enthaltend übliche Backemulgatoren sowie wenigstens ein Backenzym, dadurch gekennzeichnet, daß es als Backenzym eine Bakterien-Xylanase gemäß Anspruch 1 oder 2 enthält.

13. Backverfahren zur Herstellung von Brot und Backwaren aus Mehl, Wasser und weiteren üblichen Backhilfsmitteln durch Bereiten und Backen eines Teiges, dadurch gekennzeichnet, daß in den Teig eine Bakterien-Xylanase gemäß Anspruch 1 oder 2 eingearbeitet wird.

## Claims

1. Bacterial xylanase, characterised in that it may be prepared through expression of the structural gene according to SEQ ID NO:2.

2. Bacterial xylanase, characterised in that it may be prepared through expression of the genome of the plasmid which is contained in the Bacillus subtilis, Deposit No. DSM 7147.

3. Plasmid pIK 91, contained in the Bacillus subtilis with the Deposit No. DSM 7147, in which the structural gene of SEQ ID NO:2, the associated leader peptide sequence as well as the DNA sequences required for gene expression in Bacillus subtilis are coded.

4. A strain of bacteria, especially of the Bacillus subtilis kind, producing bacterial xylanase,
characterised by the content of a structural gene according to SEQ ID NO:2.

5. A strain of bacteria, especially of the Bacillus subtilis kind, producing bacterial xylanase,
characterised by the content of a plasmid according to Claim 3.

6. A process for preparing a bacterial xylanase according to Claim 1 or 2, characterised in that a bacterium comprising the structural gene according to SEQ ID NO:2 is cultivated in a conventional manner and the formed bacterial xylanase is recovered from the filtrate of the culture.

7. A process according to Claim 6, characterised in that a bacterium comprising a plasmid according to Claim 3 is cultivated in a conventional manner and the bacterial xylanase formed is recovered from the filtrate of the culture.

8. A process according to Claim 6 or 7, characterised in that a bacterium of the Bacillus species is used.

9. A process according to Claim 8, characterised in that a bacterium of the Bacillus subtilis type is used.

10. A process according to Claim 9, characterised in that the Bacillus subtilis with the Deposit No. DSM 7147 is used.

11. Use of the bacterial xylanase according to Claim 1 or 2 for producing bread and bakery products.

12. Baking agents for producing bread and bakery products, comprising conventional baking emulsifiers as well as at least one baking enzyme, characterised in that they comprise a bacterial xylanase according to Claim 1 or 2 as the baking enzyme.

13. A baking process for producing bread and bakery products made from flour, water and other conventional baking adjuvants, in which a dough is prepared and baked, characterised in that a bacterial xylanase according to Claim 1 or 2 is worked into the dough.

## Revendications

1. Xylanase bactérienne, caractérisée en ce qu'elle peut être préparée par expression du gène de structure selon SEQ ID NO:2.

2. Xylanase bactérienne, caractérisée en ce qu'elle peut être préparée par expression du génome du plasmide qui est contenu dans le Bacillus subtilis portant le numéro de dépôt DSM 7147.

3. Plasmide pIK 91, contenu dans le Bacillus subtilis portant le numéro de dépôt DSM 7147, sur lequel sont codés le gène de structure de SEQ ID NO: 2, la séquence de peptides de tête correspondante, ainsi que les séquences d'ADN nécessaires pour l'expression du gène dans Bacillus subtilis.

4. Souche bactérienne produisant de la xylanase bactérienne, en particulier du genre Bacillus subtilis, caractérisée par sa teneur en un gène de structure selon SEQ ID NO:2.

5. Souche bactérienne produisant de la xylanase bactérienne, en particulier du genre Bacillus subtilis, caractérisée par sa teneur en un plasmide selon la revendication 3.

6. Procédé de préparation d'une xylanase bactérienne selon la revendication 1 ou 2, caractérisé en ce que l'on cultive de la manière usuelle une bactérie qui contient le gène de structure selon SEQ ID NO:2 et on récupère, à partir du filtrat de la culture, la xylanase bactérienne formée.

7. Procédé selon la revendication 6, caractérisé en ce que l'on cultive de la manière usuelle une bactérie qui contient un plasmide selon la revendication 3 et on récupère, à partir du filtrat de la culture, la xylanase bactérienne formée.

8. Procédé selon la revendication 6 ou 7, caractérisé en ce que l'on utilise une bactérie de l'espèce Bacillus.

9. Procédé selon la revendication 8, caractérisé en ce que l'on utilise une bactérie du genre Bacillus subtilis.

10. Procédé selon la revendication 9, caractérisé en ce que l'on utilise le Bacillus subtilis portant le numéro de dépôt DSM 7147.

11. Utilisation de la xylanase bactérienne selon la revendication 1 ou 2 pour la fabrication de pain et d'articles de boulangerie-patisserie.

12. Agent de boulangerie pour la fabrication de pain et d'articles de boulangerie-patisserie contenant des émulsifiants de boulangerie usuels, ainsi qu'au moins une enzyme de boulangerie, caractérisé en ce qu'il contient, en tant qu'enzyme de boulangerie, une xylanase bactérienne selon la revendication 1 ou 2.

13. Procédé de boulangerie pour la fabrication de pain et d'articles de boulangerie-patisserie à partir de farine, d'eau et d'autres adjuvants de boulangerie usuels, par préparation et cuisson d'une pâte, caractérisé en ce qu'une xylanase bactérienne selon la revendication 1 ou 2 est incorporée dans la pâte.
